(19) Europäisches Patentamt
European Patent Office
Office européen des brevets

(11) EP 3 089 053 A1

(12) EUROPEAN PATENT APPLICATION
published in accordance with Art. 153(4) EPC

(43) Date of publication:
02.11.2016 Bulletin 2016/44

(51) Int Cl.:
G06F 17/30 (2006.01)

(21) Application number: 15832872.4

(22) Date of filing: 30.01.2015

(86) International application number:
PCT/JP2015/052777

(87) International publication number:
WO 2016/121127 (04.08.2016 Gazette 2016/31)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: Ubic, Inc.
Minato-ku, Tokyo 108-0075 (JP)

(72) Inventors:
• MORIMOTO, Masahiro
  Minato-ku,
  Tokyo 1080075 (JP)

• TAKEDA, Hideki
  Minato-ku,
  Tokyo 1080075 (JP)
• HALSKOV, Jakob
  Minato-ku,
  Tokyo 1080075 (JP)

(74) Representative: Hoefer & Partner Patentanwälte
mbB
Pilgersheimer Straße 20
81543 München (DE)

(54) DATA EVALUATION SYSTEM, DATA EVALUATION METHOD, AND DATA EVALUATION PROGRAM

(57) A data evaluation system includes: an acquisition unit that acquires, as training data, data including information representing an emotion of a user and classification information for classifying the emotion; an emotion evaluation unit that determines a degree indicating how much a data element included in the training data reflects the user's emotion, as emotion evaluation information, on the basis of the classification information; a storage unit that associates the data element with the emotion evaluation information determined for the data element and stores them in a memory unit; and an unknown data evaluation unit that evaluates an emotion of a user who has created unknown data, on the basis of the emotion evaluation information stored in the memory unit when new data is acquired as the unknown data.

FIG.1

## Description

TECHNICAL FIELD

The present invention relates to a data evaluation system, data evaluation method, and data evaluation program for analyzing data.

BACKGROUND ART

In recent years, there is numerous and abundant information and we have many opportunities to access various kinds of information. Particularly, we often access to various kinds of information via web browsing. Consequently, it has become difficult for users to search for useful information from among an enormous amount of information. So, if it is possible to estimate what would be the users' general impressions about numerous information, that can be an index to estimate whether the users will access the information or not.

An example of such estimation technology is PTL 1. PTL 1 discloses that object words which co-occur with four emotional expressions such as "happy," "sad," "angry," and "pleased" are selected in text data and weight values for the selected words are calculated; and also discloses that the text data is evaluated by using the weight values of the relevant object words.

CITATION LIST

PATENT LITERATURE

PTL 1: Japanese Patent Application Laid-Open (Kokai) Publication No. 2007-18234

SUMMARY OF INVENTION

PROBLEMS TO BE SOLVED BY THE INVENTION

However, the method described in the above-mentioned PTL 1 has a problem, that is, an inferred result of an impression which is different from a general impression what users may possibly have may sometimes be output. Therefore, in light of the above-described problem, it is an object of the present invention to provide, for example, a data evaluation system capable of estimating what would be a user's impression.

MEANS FOR SOLVING THE PROBLEMS

In order to solve the above-described problem, a data evaluation system according to an embodiment of the present invention includes: an acquisition unit that acquires, as training data, data including information representing an emotion of a user and classification information for classifying the emotion; an emotion evaluation unit that determines a degree indicating how much the user's emotion is reflected in a data element included in the training data, as emotion evaluation information, on the basis of the classification information; a storage unit that associates the data element with the emotion evaluation information determined for the data element and stores them in a memory unit; and an unknown data evaluation unit that evaluates an emotion of a user who has created unknown data, on the basis of the emotion evaluation information stored in the memory unit when new data is acquired as the unknown data.

Furthermore, a data evaluation method according to an embodiment of the present invention is a data evaluation method executed by a computer, comprising: an acquisition step of acquiring, as training data, data including information representing an emotion of a user and classification information for classifying the emotion; an emotion evaluation step of determining a degree indicating how much a data element included in the training data reflects the user's emotion, as emotion evaluation information, on the basis of the classification information; a storage step of associating the data element with the emotion evaluation information determined for the data element and storing them in a memory unit; and an unknown data evaluation step of evaluating an emotion of a user who has created unknown data, on the basis of the emotion evaluation information stored in the memory unit when new data is acquired as the unknown data.

Furthermore, a data evaluation program according to an embodiment of the present invention has a computer implement: an acquisition function that acquires, as training data, data including information representing an emotion of a user and classification information for classifying the emotion; an emotion evaluation function that determines a degree indicating how much a data element included in the training data reflects the user's emotion, as emotion evaluation information, on the basis of the classification information; a storage function that associates the data element with the emotion evaluation information determined for the data element and stores them in a memory unit; and an unknown data evaluation function that evaluates an emotion of a user who has created unknown data, on the basis of the emotion evaluation information stored in the memory unit when new data is acquired as the unknown data.

Furthermore, the emotion evaluation unit may determine the degree as the emotion evaluation information for the data element on the basis of frequency at which the data element appears in the training data classified into a specified emotion, and frequency at which the data element appears in the training data that is not classified into the specified emotion.

Furthermore, the unknown data evaluation unit may extract the data element from the unknown data, acquire the emotion evaluation information associated with the extracted data element from the memory unit, and evaluate the emotion of the user, who has created the unknown data, on the basis of the acquired emotion evaluation information.

Furthermore, the unknown data evaluation unit may further evaluate the emotion of the user who has created the unknown data on the basis of frequency at which the data element appears in the unknown data, and the emotion evaluation information associated with the data element.

Furthermore, when the data element extracted from the unknown data is modified with an exaggerated expression, the unknown data evaluation unit may evaluate the emotion of the user who has created the unknown data by enhancing the degree, wherein the degree is indicated by the emotion evaluation information associated with the data element.

Furthermore, when the data element extracted from the unknown data is modified with a negative expression, the unknown data evaluation unit may evaluate the emotion of the user who has by reducing the degree, wherein the degree is indicated by the emotion evaluation information associated with the data element. Furthermore, the data evaluation system may further include a presentation unit that presents evaluation information about the user's emotion evaluated by the unknown data evaluation unit.

Furthermore, the unknown data is an e-mail; and when the e-mail is acquired as the unknown data, the unknown data evaluation unit may evaluate the emotion of a user, who has written the e-mail, on the basis of the emotion evaluation information stored in the memory unit.

Furthermore, the unknown data is an e-mail; and the data evaluation system may further include an estimation unit that estimates a human relationship between a user who has written the e-mail and another user designated as an addressee of the e-mail on the basis of the user's emotion evaluated by the unknown data evaluation unit.

Furthermore, the unknown data is data included in a website; and when data included in the website is acquired as the unknown data, the unknown data evaluation unit may evaluate the emotion of a user, who has created the data included in the website, on the basis of the emotion evaluation information stored in the memory unit.

ADVANTAGEOUS EFFECTS OF INVENTION

The data evaluation system, the data evaluation method, and the data evaluation program according to an embodiment of the present invention can estimate the emotion of the user who has created data.

BRIEF DESCRIPTION OF DRAWINGS

[Fig. 1] Fig. 1 is a block diagram illustrating a functional configuration of a data evaluation system according to an embodiment;
[Fig. 2] Fig. 2 illustrates an example of the structure of a web page to which reference is made upon data evaluation according to an embodiment;
[Fig. 3] Fig. 3 is a flowchart illustrating processing for creating training data for data analysis; and
[Fig. 4] Fig. 4 is a flowchart illustrating processing for evaluating emotions of a user who has created unclassified data.

DESCRIPTION OF EMBODIMENTS

<Embodiment>

An embodiment of a data evaluation system according to the present invention will be described with reference to drawings.

<Outline>

The data evaluation system according to this embodiment estimates what kind of emotion (for example, a good impression or a bad impression) a user who created unknown data (mainly indicating document data [data at least partly including texts such as e-mails, presentation materials, spreadsheet materials, meeting materials, agreements, organization charts, and business plans], but including a wide variety of arbitrary data such as image data, voice data, and video data) had, on the basis of remarks (training data) that the user has made with respect to products, films, programs, and so on.

Generally, at online product sites and restaurant guides, evaluation values given by users with respect to products as well as the users' comments are often displayed.

So, inventors have thought of estimating, for example, whether users had a good impression or bad impression with respect to a certain product by creating training data on the basis of these comments and evaluations and evaluating unknown data on the basis of the training data. Specifically speaking, the inventors have come to think of identifying data elements included in high evaluation comments and data elements included in low evaluation comments and determining evaluation values of the respective data elements, thereby setting an index for evaluating new data (unknown data).

This is based on thoughts of the inventors who noticed that, for example, in a case of text data, common words (such as "good" and "fun") are often used in a plurality of high evaluation comments and different common words (such as "bad" and "boring") are often used in a plurality of low evaluation comments.

Then, the inventors have also noticed that what kind of emotion the user had when creating the new data (unknown data) can be easily estimated by using words representing emotional expressions (such as adjectives, adjective verbs, and adverbs) as the above-described words (data elements).

Regarding the data evaluation system according to the present invention, a method for selecting words used for evaluation and determining their evaluation values and a method for evaluating new data by using the evaluation values will be described below in detail.

<Configuration>

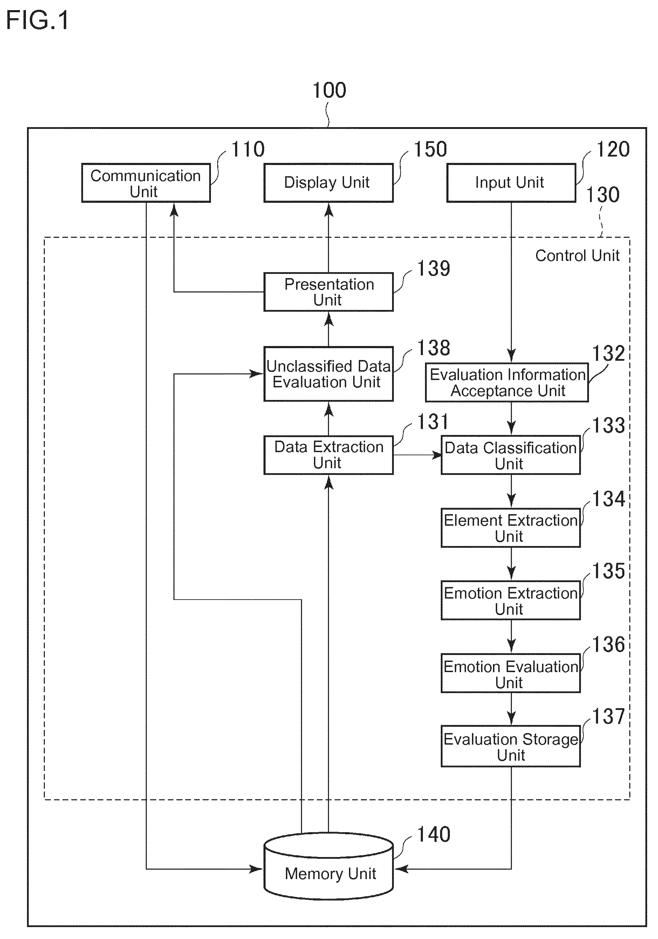

Fig. 1 is a block diagram illustrating a functional configuration of a data evaluation system 100. The data evaluation system 100 includes a communication unit 110, an input unit 120, a control unit 130, a memory unit 140, and a display unit 150 as illustrated in Fig. 1.

The communication unit 110 has a function that executes communications with external devices via a network. The communication unit 110 has a function that accesses a web page where evaluations and their relevant comments (data included in the website) are placed, collects information on the relevant web page, and stores it in the memory unit 140. Furthermore, the communication unit 110 also has a function that transmits result information transmitted from the control unit 130 (information indicating whether evaluation object data gives a good impression or a bad impression) to a user terminal when it is possible to establish communications with the user terminal.

The input unit 120 has a function that accepts input from the user and accepts input of the evaluations and comments to the web page. The input unit 120 transmits the content of the accepted input to the control unit 130.

The control unit 130 is a processor having a function that controls each unit of the data evaluation system 100 with reference to various kinds of data stored in the memory unit 140. The control unit 130 controls various functions of the data evaluation system 100 in an integrating manner.

The control unit 130 includes a data extraction unit 131, an evaluation information acceptance unit 132, a data classification unit 133, an element extraction unit 134, an emotion extraction unit 135, an emotion evaluation unit 136, an evaluation storage unit 137, an unclassified data evaluation unit 138, and a presentation unit 139.

The data extraction unit 131 has a function that extracts data from a group of information relating to the web page, which is stored in the memory unit 140, as the need arises. The data extraction unit 131 transmits classification data including the evaluations and comments corresponding to the evaluations stored in the memory unit 140 to the data classification unit 133. Furthermore, the data extraction unit 131 acquires data, which has not been evaluated, from the memory unit 140 and transmits it to the unclassified data evaluation unit 138.

The evaluation information acceptance unit 132 has a function that accepts the user's evaluation and comments about a certain object from the input unit 120 and transmits them to the data classification unit 133. Under this circumstance, any object may be used as long as it can be an object to be reviewed and may be, for example, any kind of products, foods, or programs.

The data classification unit 133 has a function that classifies the classification data accepted from the data extraction unit 131. Under this circumstance, the data classification unit 133 classifies the classification data on the basis of the evaluations included in the classification data. Specifically speaking, the classification data is evaluated in a scale of one to five according to the number of star marks; and as the number of star marks is larger, the relevant data is highly evaluated, that is, the user had a good impression about an object of the relevant classification data. Then, the data classification unit 133 classifies the classification data, regarding which the number of the star marks is 4 or 5, "highly-evaluated (good impression)"; and it classifies the classification data, regarding which the number of the star marks is 1 or 2, as "lowly-evaluated (bad impression)." The data classification unit 133 classifies the relevant data by, for example, associating classification information (flag information) indicative of classification of the classification data with the data.

The element extraction unit 134 has a function that extracts data elements from the classification data associated with the classification information by the data classification unit 133. Under this circumstance, for example, (1) if the data is document data, the element extraction unit 134 can extract key words (so-called morphemes), sentences, paragraphs, and so on included in the relevant document data as the data elements; (2) if the data is voice data, the element extraction unit 134 can extract partial voices included in the relevant voice data as the data elements; (3) if the data is image data, the element extraction unit 134 can extract partial images included in the relevant image data as the data elements; and (4) if the data is video data, the element extraction unit 134 can extract frame images (or a combination of a plurality of frame images) included in the relevant video data as the data elements.

Incidentally, the element extraction unit 134 determines the data elements to be extracted in accordance with specified selection standards. If the data is the document data, the element extraction unit 134 may extract the data elements by using so-called morpheme analysis. Furthermore, the element extraction unit 134 can also extract data elements designated by the user via the input unit 120. The element extraction unit 134 transmits the extracted data elements to the emotion extraction unit 135.

The emotion extraction unit 135 has a function that extracts data elements indicative of emotional expressions from among the transmitted data elements. Under this circumstance, adjectives, adjective verbs, and adverbs are used as the data elements indicative of the emotional expressions. It should be noted that any parts of speech other than the above-listed parts of speech may be used. The emotion extraction unit 135 transmits the extracted data elements indicative of the emotional expressions to the emotion evaluation unit 136.

The emotion evaluation unit 136 generates an emotion marker (emotion evaluation information) for the data elements (for example, morphemes that are adjectives or adjective verbs). The emotion marker is a value that serves as an index to indicate whether the user has a good impression or a bad impression. In other words, it can be said that the emotion marker indicates a degree indicating how much the user's emotion is reflected in the relevant data element. The emotion evaluation unit 136 generates the emotion marker as described below.

The emotion evaluation unit 136 firstly counts the number of times $A_F$ that a data element relating to a certain emotional expression (hereinafter referred to as data element A) appears in one or more pieces of classification data which are classified by the data classification unit 133 as expressing a good impression (that is, the classification data regarding which the number of the star marks is 4 or 5). Then, the emotion evaluation unit 136 calculates frequency $RF_P$ at which the above-mentioned data element A appears in all pieces of the classification data judged as expressing the good impression.

The relevant frequency $RF_P$ can be calculated according to the following mathematical expression (1).
[Math. 1]

$$RF_P = \frac{A_F}{N_P} \qquad \cdots (1)$$

In the above expression (1), $N_P$ represents a total number of data elements included in the one or more pieces of classification data of the good impression to be used for the judgment.

Next, the emotion evaluation unit 136 counts the number of times AN that the above-mentioned data element A appears in one or more pieces of classification data judged as expressing a bad impression (that is, the classification data regarding which the number of the star marks is 1 or 2). Then, the emotion evaluation unit 136 calculates frequency $RF_N$ at which the above-mentioned data element A appears in all pieces of the classification data judged as expressing the bad impression.

The relevant frequency can be calculated according to the following mathematical expression (2).
[Math. 2]

$$RF_N = \frac{A_N}{N_N} \qquad \cdots (2)$$

In the above expression (2), $N_N$ represents a total number of data elements included in the one or more pieces of classification data of the bad impression to be used for the judgment.

The emotion evaluation unit 136 generates the emotion marker of data element A by using the frequencies calculated by using expression (1) and expression (2). Specifically speaking, the emotion evaluation unit 136 calculates an emotion judgment index value P(A) by using the following mathematical expression (3).
[Math. 3]

$$P(A) = \frac{RF_P}{RF_N} \qquad \cdots (3)$$

Then, when the emotion judgment index value P(A) is more than 1, the emotion evaluation unit 136 determines data element A as a data element, which is often used in data expressing the good impression, and designates "+1" as its emotion marker; and when the emotion judgment index value P(A) is less than 1, the emotion evaluation unit 136 determines data element A as a data element, which is often used in data expressing the bad impression, and designates "-1" as its emotion marker and transmits it to the evaluation storage unit 137.

As a result, the memory unit 140 stores: "+1" as the emotion marker for words often used in documents of the good impression; and "-1" as the emotion marker often used in documents of the bad impression. For example, words such as "good," "beautiful," and "taste good" tend to get "+1," while words such as "bad," "dirty," and "taste bad" tend to get "-1." The emotion evaluation unit 136 transmits an evaluation value and threshold value of each calculated data element to the evaluation storage unit 137.

The evaluation storage unit 137 has a function that associates each data element evaluated by the emotion evaluation unit 136 with its evaluation and stores them in the memory unit 140.

The unclassified data evaluation unit 138 has a function that estimates whether input data, regarding which whether it relates to the good impression or the bad impression is unknown (hereinafter referred to as the "unclassified data"), relates to the good impression or the bad impression.

The unclassified data evaluation unit 138 extracts the data elements from the unclassified data. Then, the unclassified data evaluation unit 138 extracts data elements relating to emotional expressions from the above-extracted data elements. Specifically speaking, the unclassified data evaluation unit 138 extracts the data elements to which emotion markers are set in the memory unit 140.

Then, the unclassified data evaluation unit 138 acquires emotion marker values of the respective extracted data elements from the memory unit 140.

The unclassified data evaluation unit 138 acquires the emotion markers of the relevant data elements and adds the emotion marker values as many as the number of times of appearance in the unclassified data. For example, when the emotion marker which is set to a data element "good" is "+1" and appears five times in the unclassified data, an emotion score based on the data element "good" in the unclassified data is set to "5." Furthermore, for example, when the emotion marker which is set a data element "bad" is "-1" and appears three times in the unclassified data, the emotion score based on the data element "bad" in the unclassified data is set to "-3."

Under this circumstance, the unclassified data evaluation unit 138 judges whether a negative expression or an exaggerated expression modifies the data element or not; and if the negative expression or the exaggerated expression modifies the data element, the unclassified data evaluation unit 138 applies the following processing and then calculate the emotion score.

The negative expression is an expression to deny the data element, for example, an expression such as "not good" or "do not taste good." When such an expression exists, it is treated as an opposite expression. For example, if the expression is "not good," it is treated as "bad"; and if the expression is "do not taste good," it is treated as "taste bad." Incidentally, it is decided under this circumstance to treat the above-described negative expression as the opposite expression; however, for example, when the emotion marker "+1" is set to the expression "good," this may be changed to a negative value. Alternatively, a value which is set as the emotion marker may be reduced by a specified value (for example, 1.5). Furthermore, whether an expression to deny negation, that is, a double negative expression exists or not is detected; and if the double negative expression exists, the data element may be judged to be affirmative.

Furthermore, the exaggerated expression is an expression to exaggerate (or emphasize) the data element more and indicates an expression such as "very," "so," or "much." If the above-described exaggerated expression modifies the data element, the emotion score is calculated by multiplying its emotion marker value by a specified number (for example, by doubling the emotion marker value). For example, when the expression "taste very good" exists and the emotion marker value of "taste good" is "+1," the emotion score of this expression is set (or increased) to "+2". It should be noted that the data element to be multiplied by the specified number is only the data element modified by the exaggerated expression.

Accordingly, the unclassified data evaluation unit 138 calculates data score S of the unclassified data by calculating and summing up emotion scores based on all the data elements as shown in the following mathematical expression (4).
[Math. 4]

$$S = \sum_{i=1}^{N} s_i \qquad \cdots (4)$$

$S_i$ is an emotion marker for an i-th data element.

Then, when the data score is more than 0, the unclassified data evaluation unit 138 estimates that the unclassified data is data which tends to give a good impression; and when the data score is less than 0, the unclassified data evaluation unit 138 estimates that the unclassified data is data which tends to give a bad impression. When the data score is 0, the unclassified data evaluation unit 138 judges that the unclassified data tends to give neither the good impression nor the bad impression. The unclassified data evaluation unit 138 transmits evaluation obtained by the estimation (estimation as to whether the data tends to give the good impression or the bad impression) to the presentation unit 139.

The presentation unit 139 has a function that presents result information from the unclassified data evaluation unit 138, indicating whether the unclassified data is data which tends to give the good impression or the bad impression. The presentation unit 139 transmits the result information via the communication unit 110 to a user terminal or the display unit 150.

The memory unit 140 is a storage medium having a function that stores necessary programs and various kinds of data to be used by the data evaluation system 100 to analyze the data. The memory unit 140 is implemented by, for example, HDDs (Hard Disc Drives), SSDs (Solid State Drives), semiconductor memories, or flash memories. It should be noted that Fig. 1 illustrates the configuration of the data evaluation system 100 equipped with the memory unit 140, but the memory unit 140 may be a storage device outside the data evaluation system 100 and connected to the data evaluation system 100 so that they can communicate with each other.

The display unit 150 is a monitor having a function that displays images based on display data which is output from the control unit 130. The display unit 150 may be implemented by, for example, an LCD (Liquid Crystal Display), a PDP (Plasma Display Panel), or an organic EL (Electro Luminescence) display. In this embodiment, the display unit 150 displays the result information transmitted from the presentation unit 139.

<Web Page>

Now, a web page will be briefly explained below.

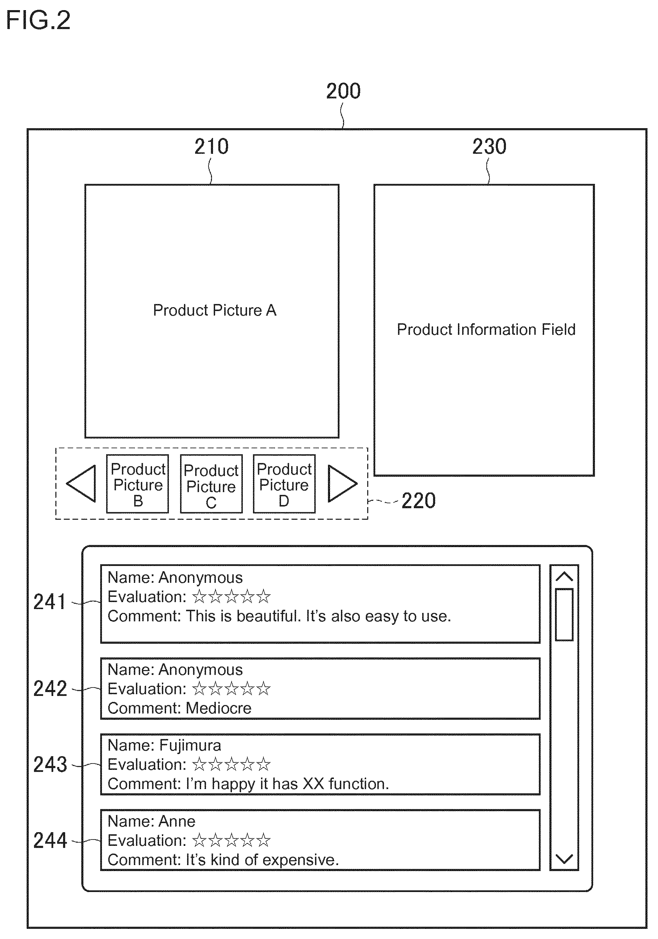

Fig. 2 is a diagram illustrating an example of the structure of the web page and shows a page on which a plurality of users have input their evaluations and comments. A web page 200 of Fig. 2 is a page example of an online shopping site. The web page 200 illustrated in Fig. 2 includes a product picture A210, a product picture group 220, a product information field 230, and comments 241 to 244.

The product picture A210 is an appearance picture taken of a product.

The product picture group 220 is thumbnails of appearance pictures taken of the product from different angles. If you click on the relevant thumbnail, the selected picture will be displayed in the area where the appearance picture A210 is displayed.

The product information field 230 is used to describe, for example, the price and size of the product.

The comments 241 to 244 are information written about impressions that users who saw or used the product had.

Each comment 241 to 244 includes the name of a user who wrote the information, and the evaluation and impression of the product by the user as shown in Fig. 2. In this example, the evaluation is expressed with star marks and five-level evaluation is performed. A larger number of the star marks mean that the relevant object (product) is highly-evaluated (good impression).

Each one of these comments is treated as the classification data in this embodiment.

It should be noted that the structure of the web page illustrated in Fig. 2 is just one example and it is needless to say that there are various forms of structures of web pages.

<Operation>

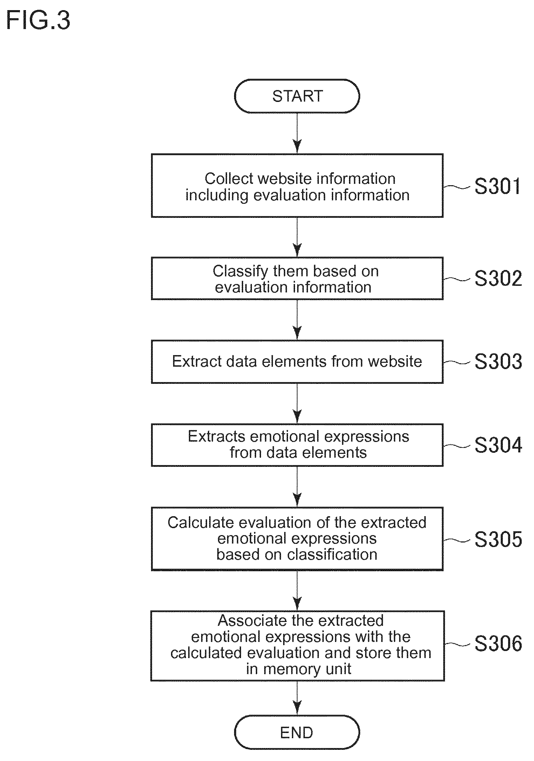

Fig. 3 is a flowchart illustrating operation of the data evaluation system 100 to analyze the classification data on web pages including evaluations and comments and calculate the evaluations of the data elements indicating the emotional expressions.

Referring to Fig. 3, the data extraction unit 131 for the data evaluation system 100 collects web pages including evaluations and comments as the classification data from the memory unit 140 (step S301).

Next, the data classification unit 133 for the data evaluation system 100 classifies whether the relevant classification data is data of a good impression or not, on the basis of the evaluations included in the classification data (step S302).

The element extraction unit 134 extracts data elements from the classification data (step S303).

The emotion extraction unit 135 extracts data elements indicative of emotional expressions from the data elements extracted by the element extraction unit 134 (step S304).

The emotion evaluation unit 136 evaluates each of the data elements indicative of the emotional expressions extracted by the emotion extraction unit 135 and transmits their evaluation values to the evaluation storage unit 137 (step S305). The evaluation storage unit 137 associates the transmitted data elements with their evaluation values and stores them in the memory unit 140 (step S306).

The operation of the data evaluation system 100 to determine each of the evaluations of the data elements has been described above. The processing illustrated in Fig. 3 is processing for acquiring evaluations (classification information) and comments, which are given by various users about an object, as training data and evaluating data elements included in the training data in order to classify whether the unclassified data is data which tends to give a good impression, or data which tends to give a bad impression. By executing the processing illustrated in Fig. 3, preprocessing for identifying web pages, which can be estimated to attract interests of the user, from among web pages which the user has never accessed is completed.

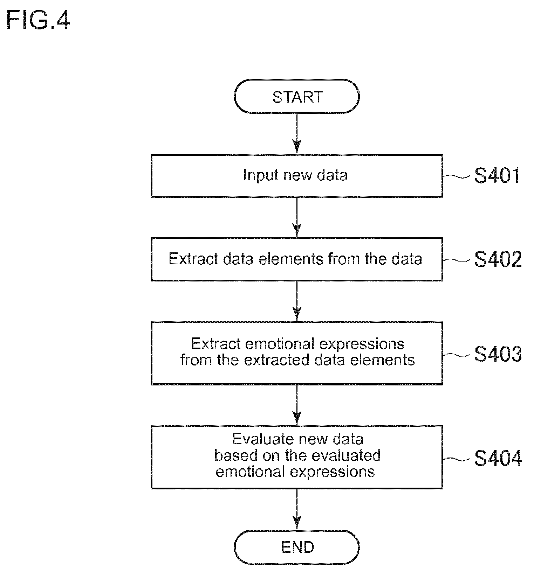

Fig. 4 is a flowchart illustrating operation of the data evaluation system 100 to classify the unclassified data regarding which whether it is data of the good impression or data of the bad impression has not been classified.

The input unit 120 or the communication unit 110 for the data evaluation system 100 accepts data, regarding which whether it gives the good impression or the bad impression has not been classified, as new data (step S401). The relevant data is stored in the memory unit 140.

After the unclassified data evaluation unit 138 accepts the unclassified data stored in the memory unit 140 from the data extraction unit 131, it extracts data elements from the relevant unclassified data (step S402).

The unclassified data evaluation unit 138 extracts data elements (adjectives, adjective verbs, and adverbs in this example) indicative of emotional expressions from the extracted data elements (step S403).

The unclassified data evaluation unit 138 acquires emotion markers of the extracted data elements indicative of the emotional expressions from the memory unit 140.

Then, the unclassified data evaluation unit 138 calculates a score of the unclassified data on the basis of the acquired emotion markers in consideration of the number of appearances of each data element, negative expressions and exaggerated expressions. Then, when the calculated score indicates a positive value, the unclassified data evaluation unit 138 generates result information indicating that the relevant unclassified data is data which tends to give the good impression; and when the calculated score indicates a negative value, the unclassified data evaluation unit 138 generates the result information indicating that the relevant unclassified data is data which tends to give the bad impression (step S404).

The generated result information is output by the presentation unit 139 to the communication unit 110 or the display unit 150 and is presented to the user.

By executing the processing illustrated in Fig. 4, the data evaluation system 100 can estimate whether the unclassified data is (affirmative) data of the good impression or (negative) data of the bad impression.

<Conclusion>

By executing the above-described processing, the data evaluation system 100 can evaluate whether input data is data of the good impression (affirmative) or data of the bad impression (negative). Therefore, even if the detailed content of the data is unknown, the user can imagine the content of the data. Furthermore, since evaluations and comments which have already been made on web pages are used as data to be used to classify the unclassified data, that is, as the training data, objective opinions can be treated as the training data. Therefore, an operator of the data evaluation system 100 does not have to handle cumbersome process to judge whether the data is affirmative or negative, and input the judgment result. Furthermore, since opinions of many general users are used, a general and versatile model (emotion marker) can be created.

<Variations>

Embodiment 1 above has described an embodiment of the invention according to the present invention; however, it is needless to say that the concept of the present invention is not limited to this embodiment. Various kinds of variations included as the concept of the present invention will be explained below.

(1) In the above-described embodiment, the emotion marker is set to "+1" in an affirmative case and "-1" in a negative case; however, the invention is not limited to this example.
Specifically speaking, the value of the emotion marker may be weighted or unweighted with respect to the data elements.
For example, the value of the emotion marker may be weighted or unweighted according to the frequency at which the relevant data element appears in the classification data. The value of the emotion marker may be increased (to, for example, 1.8) with respect to a data element which often appears, while the value of the emotion marker may be reduced (to, for example, 0.5) with respect to a data element which rarely appears.
(2) In the above-described embodiment, the unclassified data evaluation unit 138 evaluates the unclassified data by calculating the total sum of the emotion marker values of the data elements indicative of the emotional expressions; however, the invention is not limited to this example.
For example, the score of the unclassified data may be calculated by generating vectors whose elements are emotion marker values of data elements, generating a vector indicative of the number of extracted data elements relating to emotional expressions from the unclassified data, and calculating an inner product of these vectors.
Alternatively, the unclassified data evaluation unit 138 may calculate score S of the unclassified data by placing emphasis on appearance frequency of the data elements by using the following expression (5).
[Math. 5]

$$S = \frac{\sum_{j=1}^{N} m_j w_j^2}{\sum_{i=1}^{N} w_i^2} \qquad \cdots (5)$$

In the above expression, $m_j$ represents the appearance frequency of a j-th keyword and $w_i$ represents an emotion marker value of a data element relating to an i-th emotional expression.
(3) Although the aforementioned embodiment does not include detailed explanations, the unclassified data evaluation unit 138 may calculate a score based on co-occurrence between the data elements. The details of such a method will be explained below.
For example, it is assumed that a first keyword and a second keyword appear as data elements relating to emotional expressions on a web page which is an object to be evaluated. Under this circumstance, when the first keyword appears on the web page, the unclassified data evaluation unit 138 may execute scoring in consideration of the appearance frequency of the second keyword on the relevant web page (which may also be referred to as the correlation or co-occurrence between the first keyword and the second keyword).
In this case, the unclassified data evaluation unit 138 may calculate the score by using correlation matrix (co-occurrence matrix) C representing the correlation (co-occurrence) between the first keyword and the second keyword according to the following expression (6) instead of the aforementioned expression (2).
[Math. 6]

$$S = \mathbf{w}^T \cdot (\boldsymbol{C} \cdot \boldsymbol{s}) \qquad \cdots (6)$$

It should be noted that the above correlation matrix C is optimized in advance by using learning data which includes a specified number of specified texts. Furthermore, matrix w is a matrix indicating emotion marker values. For example, when the keyword "fun" appears in a certain text, a value obtained by normalizing the number of appearances of other keywords relative to the relevant keyword between 0 and 1 (which may also be referred to as the maximum likelihood estimate) is stored in an element of the above-mentioned correlation matrix C.
Since the score in consideration of the correlation between the keywords can be calculated by using the expression (6), it is possible to estimate a web page which may highly possibly attract the users' interests with high precision.
(4) In the aforementioned embodiment, web page information is used as data which is an emotion evaluation object; however, the invention is not limited to this example. A data group which is an object to be classified may be, for example, a mail data group, a medical record data group, or a lawsuit-related data group.

(5) The aforementioned embodiment has described an example of analyzing document information (texts); however, voices, images, and videos may be analyzed as mentioned earlier.

For example, in a case of voices, voices themselves may be objects to be analyzed or the analysis may be performed after converting voices into documents by means of voice recognition.

When a voice itself is to be analyzed, the voice is divided into partial voices of a specified length and the partial voices are used as objects to be analyzed. For example, if a voice stating "this film is interesting" is obtained, the data evaluation system 100 can extract the partial voice "interesting" from the relevant voice and generate its emotion marker on the basis of the evaluation result of that partial voice. In such a case, the data evaluation system 100 can classify the voice by using chronological data classification algorithms (such as the Markov model and the Kalman filter).

When converting voices into texts, they may be classified in the same manner as indicated in the aforementioned embodiment. Arbitrary voice recognition algorithms (such as a recognition method using the hidden Markov model) may be used for conversion of the voices into the texts.

(6) Regarding objects to be evaluated by the data evaluation system 100 indicated in the aforementioned embodiment, the data evaluation system 100 can be also applied to the following objects.

For example, the data evaluation system 100 can be applied to a medical application system (a system for estimating emotions of injured and sick persons by using electronic medical records, nursing records, patients' diaries, and so on as data). In this case, the medical application system extracts the data elements indicative of the emotional expressions included in the classification data (such as electronic medical records, nursing records, and patients' diaries) and evaluates them on the basis of whether the relevant data is affirmative or negative. Under this circumstance, the user judges whether the classification data is affirmative data or negative data, and inputs the judgment result via the input unit 120.

Then, the unclassified data evaluation unit 138 can estimate a patient's mental state (for example, their mental state in which they are feeling anxious about the present condition of their injury or disease or they are worried if they will get better) on the basis of emotional expressions included in the unclassified data (such as electronic medical records, nursing records, and patients' diaries)).

Furthermore, the data evaluation system 100 can be applied to a mail monitoring system. In this case, the mail monitoring system evaluates whether the user feels, for example, dissatisfied with the content of the classification data (for example, e-mails exchanged daily on the network) (or whether they may possibly conduct any fraudulent act or not). Then, the mail monitoring system extracts data elements relating to emotional expressions from the relevant classification data on the basis of the evaluation and generates emotion markers based on whether the user feels dissatisfied or not.

Then, the unclassified data evaluation unit 138 evaluates the unclassified data (such as a new e-mail) based on the relevant emotion marker. Accordingly, for example, it is possible to estimate whether an employee who wrote the e-mail in a company has complaints about, or feels dissatisfied with, the company or not (or whether they may possibly conduct any fraudulent act) and thereby prevent any risk of the fraudulent act by the employee (such as information leakage). Furthermore, under this circumstance, by clustering the unclassified data evaluated as the person who created the unclassified data having complaints or feeling dissatisfied, in order to see regarding what the person who created the unclassified data has complaints or feels dissatisfied (for example, dissatisfied with their remuneration or dissatisfied with their labor environment), proportions of e-mails expressing complaints and dissatisfaction can be visualized, for example, as follows: "e-mails not expressing complaints or dissatisfaction: 92%; e-mails expressing dissatisfaction about the remuneration: 3%; e-mails expressing dissatisfaction about the labor environment: 2%; and others: 3%."

Furthermore, the e-mails can also be used to prepare a personal correlation diagram on the basis of the emotional expressions included in the relevant e-mails. For example, when sending e-mails from a person of a subordinate position to a person of a superior position in a certain organization, it is difficult to send e-mails containing negative content; however, it is relatively easier for the person of the superior position to send such e-mails to the person of the subordinate position. So, it is possible to estimate the hierarchical relationship between members in the organization on the basis of the results of emotion analysis and senders and addressees of e-mails. For that purpose, the data evaluation system 100 may include an estimation unit to estimate the relevant correlation. For example, the estimation unit extracts the data elements from a specified number of e-mails sent from person A to person B and detects emotions of user A, who wrote the e-mails, to check whether there are many affirmative e-mails or many negative e-mails. Then, if the estimation unit detects that there are many affirmative e-mails, it estimates that person A is subordinate to person B in terms of their positions; and if the estimation unit detects that there are many affirmative e-mails, it estimates that person A is superior to person B in terms of their positions.

Furthermore, the data evaluation system 100 can be applied to a performance evaluation system. In this case, the performance evaluation system evaluates whether the classification data (such as daily reports submitted by sales persons to a company, analysis materials submitted by consultants to clients, and user questionnaires about some kind of projects) is affirmative or negative, and evaluates the data elements indicative of the emotional expressions included in the classification data. Then, emotion analysis can be performed based on, for example, a user questionnaire at a shop as the unclassified data and the analysis result can be used as materials to judge the management situation of the shop (for example, whether customers are dissatisfied with shop clerks' attitude in helping and taking care of the customers, and whether they are satisfied with how products are displayed).

Furthermore, the data evaluation system 100 can also be applied to an intellectual property evaluation system, a marketing support system, a driving support system, and so on.

Furthermore, the data evaluation system 100 can also be applied to a discovery support system. The discovery support system may perform emotion analysis of a plurality of e-mails exchanged between objects (such as companies) and identify e-mails which are estimated as having been written with emotions relating to money (for example, cheap or expensive) in order to, for example, prevent cartels.

Furthermore, the data evaluation system 100 can also be applied to a forensic system. The forensic system can perform emotion analysis of, for example, e-mails sent and received by a suspect, identify e-mails estimated as having written with evil intent, and make use of such e-mails to identify their motive for committing a fraudulent act or whether the suspect is planning to commit any fraudulent act or not.

The above-mentioned data evaluation system can be implemented with at least three configurations described below. Specifically speaking, the above-mentioned data evaluation system may be implemented with: (a) a configuration in which a part or whole of a data analysis program for implementing the relevant data evaluation system is executed at a client device (for example, a user terminal such as a personal computer or a smart phone); (b) a configuration in which a part or whole of the above-mentioned data analysis program is executed at a server apparatus (for example, a mainframe, a cluster computer, or an arbitrary computer capable of providing services by the above-mentioned system to external equipment) and the execution result is returned to the above-mentioned client device; or (c) a configuration in which processing included in the above-mentioned data analysis program is arbitrarily shared by the above-mentioned client device and the server apparatus. In other words, all that is required is that the above-mentioned data evaluation system should be implemented as a system configured of at least one computer; and each function included in the relevant data evaluation system can be arbitrarily shared and implemented by the computer(s) constituting the system.

Accordingly, the data evaluation system according to the present invention can be applied to an arbitrary system that achieves the object, by analyzing the emotions included in various kinds of data used in various systems.

(7) When the data evaluation system described in the aforementioned embodiment extracts and evaluates the users' emotions (such as anxiety and irritation) about, for example, incidents causing a disturbance to the public (such as terrorism incidents) by analyzing the emotions based on information from SNSs and news sites as the classification data and evaluates, for example, e-mails in organizations as the unclassified data, it is possible to enhance analysis accuracy of the e-mails in the organizations by offsetting influences of these incidents with evaluations of the extracted emotions. Generally, there is a high possibility that e-mails written under the influences of the social conditions in the world may be different from those written in a normal state of mind and may thereby cause degradation of the accuracy of analysis of the e-mails; however, it is possible to prevent degradation of the analysis accuracy by applying the above-described offset.

(8) In the aforementioned embodiment, the data elements are evaluated with binary values by using evaluations on web pages (the emotion is "good" when the number of the star marks is 4 or 5; and the emotion is "bad" when the number of the star marks is 1 or 2) when evaluating the users' emotions; however, the invention is not limited to this example.

For example, the emotions may be evaluated according to five classification levels by employing: "very good" in a case of five star marks; "good" in a case of four star marks; and "average," "bad," and "very bad" in a case of three star marks.

Furthermore, other emotions such as "interesting" and "boring" or emotions such as "happy" and "sad" may be used for classification, instead of "good" and "bad," as the classification data.

Furthermore, the unclassified data evaluation unit 138 may evaluate the emotions of the user who created the unclassified data by combining the emotion markers of the data elements evaluated with "good" and "bad" and the emotion markers of the data elements evaluated with "interesting" and "boring."

(9) An example to use comments on web pages has been described as an example of the classification data and the unclassified data of the above-mentioned data evaluation system 100; however, the invention is not limited to this example. Object data of the classification data and the unclassified data may be the content of messages of messaging services, blogs on web pages, recipe information, chat content of chat systems, and data and articles exchanged in SNS's.

For example, the emotion markers for evaluating users' emotions may be created based on messages in a service for exchanging the messages between the users and the users' opinions exchanged in a chat system. Furthermore, the unclassified data evaluation unit 138 may identify a user's emotions and identify whether they have radical thoughts or not, by using the created emotion markers and on the basis of such messages and opinions and the presentation unit 139 may present information indicating that the relevant user is dangerous (Internet monitoring system).

Alternatively, if the unclassified data evaluation unit 138 analyzes a blog article and evaluates that a user who wrote that blog article wrote it with evil intent, the presentation unit 139 may present information indicating that the holder of that blog is a person with dangerous thoughts.

Alternatively, if the unclassified data evaluation unit 138 evaluates that the content of a web article include many affirmative emotions (such as fun and happy), the presentation unit 139 may present that web article as information recommended to the users. The recommended information may be about products introduced on a web page with many favorable emotions.

The data evaluation system 100 may be utilized in this manner.

(10) Each functional unit of the data evaluation system 100 (the information processing apparatus) may be implemented by a logical circuit (hardware) formed on, for example, an integrated circuit (IC chip). Each functional unit of the data evaluation system 100 may be implemented by one or more integrated circuits or a plurality of functional units may be implemented by one integrated circuit.

Alternatively, the functions implemented by the respective functional units of the data evaluation system 100 may be implemented by software by using a CPU (Central Processing Unit). In this case, the data evaluation system 100 includes, for example: a CPU for executing commands of a data evaluation program which is software for implementing each function; a ROM (Read Only Memory) or a storage device (collectively referred to as the "storage media") in which the above-mentioned game program and various kinds of data are recorded in a manner such that they can be read by the computer (or CPU); and a RAM (Random Access Memory) for expanding the above-mentioned data evaluation program. Then, the object of the present invention is achieved as the computer (or CPU) reads the above-mentioned data evaluation program from the above-mentioned storage media and executes it. As the above-mentioned storage media, "tangible media which are not temporary" such as tapes, disks, cards, semiconductor memories, or programmable logical circuits can be used. Furthermore, the above-mentioned data evaluation program may be supplied to the above-mentioned computer via an arbitrary transmission medium capable of transmitting the relevant game program (such as a communication network or a broadcast wave). The present invention can also be implemented in a form of a data signal embedded in a carrier wave in which the above-mentioned data evaluation program is embodied via electronic transmission.

It should be noted that the above-mentioned data evaluation program can be implemented by using, for example, a script language such as ActionScript or JavaScript (registered trademarks), an object-oriented programming language such as Objective-C or Java (registered trademarks), and a markup language such as HTML5. Furthermore, a distributed data evaluation system including an information processing apparatus equipped with the respective units, which implement the respective functions implemented by the above-mentioned data evaluation program, and a server equipped with the respective units which implement the remaining functions different from the above-mentioned the respective functions also falls under the category of the present invention.

(11) The present invention has been described with reference to the respective drawings and examples; however, it should be noted that a person skilled in the art could easily make various variations or modifications on the basis of this disclosure. Therefore, it should be noted that these variations and modifications are included in the scope of the present invention. For example, functions or the like included in the respective functional units, the respective steps, and so on can be relocated and it is possible to combine a plurality of means or steps into one means or step or divide them.

(12) The configurations indicated in the aforementioned embodiment and various kinds of variations may be combined as appropriate.

<Supplement>

An embodiment of the data evaluation system according to the present invention and its advantageous effects will be described below.

(a) A data evaluation system according to the present invention includes: an acquisition unit (110 or 120) that acquires, as training data (classification data), data including information representing an emotion of a user and classification information for classifying the emotion; an emotion evaluation unit (136) that determines a degree indicating how much a data element included in the training data reflects the user's emotion, as emotion evaluation information (an emotion marker), on the basis of the classification information; a storage unit (137) that associates the data element with the emotion evaluation information determined for the data element and stores them in a memory unit (140); and an unknown data evaluation unit (138) that evaluates an emotion of a user who has created unknown data (unclassified data), on the basis of the emotion evaluation information stored in the memory unit when new data is acquired as the unknown data.

Furthermore, a data evaluation method according to the present invention is a data evaluation method executed by a computer, the method including: an acquisition step of acquiring, as training data, data including information representing an emotion of a user and classification information for classifying the emotion; an emotion evaluation step of determining a degree indicating how much a data element included in the training data reflects the user's emotion, as emotion evaluation information, on the basis of the classification information; a storage step of associating the data element with the emotion evaluation information determined for the data element and storing them in a memory unit; and an unknown data evaluation step of evaluating an emotion of a user who has created unknown data, on the basis of the emotion evaluation information stored in the memory unit when new data is acquired as the unknown data.

Furthermore, a data evaluation program according to the present invention has a computer implement: an acquisition function that acquires, as training data, data including information representing an emotion of a user and classification information for classifying the emotion; an emotion evaluation function that determines a degree indicating how much a data element included in the training data reflects the user's emotion, as emotion evaluation information, on the basis of the classification information; a storage function that associates the data element with the emotion evaluation information determined for the data element and stores them in a memory unit; and an unknown data evaluation function that evaluates an emotion of a user who has created unknown data, on the basis of the emotion evaluation information stored in the memory unit when new data is acquired as the unknown data.

As a result, the data evaluation system can evaluate the emotion of the user who has created the unknown data by using the data element which represents an emotional expression. Therefore, for example, if the emotion of the user who has written e-mails exchanged as the unknown data in an organization is evaluated, it is possible to detect whether the user is dissatisfied with the organization or not.

(b) Regarding the data evaluation system according to (a) described above, the emotion evaluation unit may determine the degree as the emotion evaluation information for the data element on the basis of frequency at which the data element appears in the training data classified into a specified emotion, and frequency at which the data element appears in the training data that is not classified into the specified emotion.

As a result, the data evaluation system can determine the degree to reflect the user's emotion on the basis of the frequency at which the data element appears. It is possible to estimate that: data elements which appear frequently are closely related to the user's emotion; and data elements which rarely appear are not related to the user's emotion so much.

(c) Regarding the data evaluation system according to (a) or (b) described above, the unknown data evaluation unit may extract the data element from the unknown data, acquire the emotion evaluation information associated with the data element from the memory unit, and evaluate the emotion of the user, who has created the unknown data, on the basis of the acquired emotion evaluation information.

As a result, the data evaluation system can evaluate the emotion of the user, who has created the unknown data, on the basis of the emotion evaluation information which is associated in advance with the data element included in the unknown data.

(d) Regarding the data evaluation system according to (c) described above, the unknown data evaluation unit may further evaluate the emotion of the user who has created the unknown data on the basis of frequency at which the data element appears in the unknown data, and the emotion evaluation information associated with the data element.

When the data element associated with the emotion evaluation information appears more frequently, it is possible to assume that the degree of relation with the user's emotions is higher. Therefore, the emotions of the user who has created the unknown data can be evaluated more accurately by taking into consideration the frequency at which the data element appears in the unknown data.

(e) Regarding the data evaluation system according to (c) or (d) described above, when the data element extracted from the unknown data is modified with an exaggerated expression, the unknown data evaluation unit may evaluate the emotion of the user who has created the unknown data by enhancing the degree, wherein the degree is indicated by the emotion evaluation information associated with the data element.

When the data element from the unknown data is modified with the exaggerated expression, it is possible to assume that the degree of relation with the user's emotion is higher. Therefore, when evaluating the emotions of the user who has created the unknown data, the emotions of the user who has created the unknown data can be evaluated more accurately by taking into consideration whether the data element is modified with the exaggerated expression or not.

(f) Regarding the data evaluation system according to any one of (c) to (e) described above, when the data element extracted from the unknown data is modified with a negative expression, the unknown data evaluation unit may evaluate the emotion of the user who has created the unknown data by reducing the degree, wherein the degree is indicated by the emotion evaluation information associated with the data element.

When the data element is modified with the negative expression, it is possible to assume that the user created the unknown data, having an emotion opposite to the emotion for the data element. Therefore, when evaluating the emotions of the user who has created the unknown data, the emotion of the user who has created the unknown data can be evaluated more accurately by taking into consideration whether the data element is modified with the negative expression or not.

(g) Regarding the data evaluation system according to any one of (a) to (f) described above, the data evaluation system may further include a presentation unit that presents evaluation information about the user's emotion evaluated by the unknown data evaluation unit. As a result, the user can recognize the emotion of the user who has created the unknown data.

(h) Regarding the data evaluation system according to any one of (a) to (g) described above, the unknown data is an e-mail; and when the e-mail is acquired as the unknown data, the unknown data evaluation unit may evaluate the emotion of a user, who has written the e-mail, on the basis of the emotion evaluation information stored in the memory unit.

As a result, for example, it is possible to detect dissatisfaction with an organization and prevent possible fraudulent acts by acquiring e-mails exchanged within the organization as the unknown data and recognizing the emotion of the user who wrote each e-mail.

(i) Regarding the data evaluation system according to any one of (a) to (g) described above, the unknown data is an e-mail; and the data evaluation system may further include an estimation unit that estimates a human relationship between the user who has written the e-mail and another user designated as an addressee of the e-mail on the basis of the user's emotion evaluated by the unknown data evaluation unit.

As a result, the data evaluation system can estimate a personal correlation between the user and a person who is the addressee of the relevant e-mail, on the basis of the unknown data, that is, the user's emotion included in the e-mail. Therefore, the data evaluation system can support, for example, preparation of a personal correlation diagram.

INDUSTRIAL APPLICABILITY

The present invention can be applied to a wide variety of arbitrary computers such as personal computers, server apparatuses, workstations, and mainframes.

REFERENCE SIGNS LIST

| 100 | data evaluation system |
| 110 | communication unit |
| 120 | input unit |
| 130 | control unit |
| 131 | data extraction unit |
| 132 | evaluation information acceptance unit |
| 133 | data classification unit |
| 134 | element extraction unit |
| 135 | emotion extraction unit |
| 136 | emotion evaluation unit |
| 137 | evaluation storage unit |
| 138 | unclassified data evaluation unit (unknown data evaluation unit) |
| 139 | presentation unit |
| 140 | memory unit |
| 150 | display unit |

**Claims**

1. A data evaluation system comprising:

an acquisition unit that acquires, as training data, data including information representing an emotion of a user and classification information for classifying the emotion;
an emotion evaluation unit that determines a degree indicating how much a data element included in the training data reflects the user's emotion, as emotion evaluation information, on the basis of the classification information;
a storage unit that associates the data element with the emotion evaluation information determined for the data element and stores them in a memory unit; and
an unknown data evaluation unit that evaluates an emotion of a user who has created unknown data, on the basis of the emotion evaluation information stored in the memory unit when new data is acquired as the unknown data.

2. The data evaluation system according to claim 1, wherein the emotion evaluation unit determines the degree as the emotion evaluation information for the data element on the basis of frequency at which the data element appears in the training data classified into a specified emotion, and frequency at which the data element appears in the training data that is not classified into the specified emotion.

3. The data evaluation system according to claim 1 or 2, wherein the unknown data evaluation unit extracts the data element from the unknown data, acquires the emotion evaluation information associated with the extracted data element from the memory unit, and evaluates the emotion of the user, who has created the unknown data, on the basis of the acquired emotion evaluation information.

4. The data evaluation system according to claim 3, wherein the unknown data evaluation unit further evaluates the emotion of the user who has created the unknown data on the basis of frequency at which the data element appears in the unknown data, and the emotion evaluation information associated with the data element.

5. The data evaluation system according to claim 3 or 4, wherein when the data element extracted from the unknown data is modified with an exaggerated expression, the unknown data evaluation unit evaluates the emotion of the user who has created the unknown data by enhancing the degree, wherein the degree is indicated by the emotion evaluation information associated with the data element.

6. The data evaluation system according to any one of claims 3 to 5, wherein when the data element extracted from the unknown data is modified with a negative expression, the unknown data evaluation unit evaluates the emotion of the user who has created the unknown data by reducing the degree, wherein the degree is indicated by the emotion evaluation information associated with the data element.

7. The data evaluation system according to any one of claims 1 to 6, further comprising a presentation unit that presents evaluation information about the user's emotion evaluated by the unknown data evaluation unit.

8. The data evaluation system according to any one of claims 1 to 7, wherein the unknown data is an e-mail; and wherein when the e-mail is acquired as the unknown data, the unknown data evaluation unit evaluates the emotion of a user, who has written the e-mail, on the basis of the emotion evaluation information stored in the memory unit.

9. The data evaluation system according to any one of claims 1 to 7, wherein the unknown data is an e-mail; and wherein the data evaluation system further comprises an estimation unit that estimates a human relationship between a user who has written the e-mail and another user designated as an addressee of the e-mail on the basis of the user's emotion evaluated by the unknown data evaluation unit.

10. The data evaluation system according to any one of claims 1 to 7, wherein the unknown data is data included in a website; and wherein when the data included in the website is acquired as the unknown data, the unknown data evaluation unit evaluates the emotion of a user, who has created the data included in the website, on the basis of the emotion evaluation information stored in the memory unit.

11. A data evaluation method executed by a computer, comprising:

an acquisition step of acquiring, as training data, data including information representing an emotion of a user and classification information for classifying the emotion; an emotion evaluation step of determining a degree indicating how much a data element included in the training data reflects the user's emotion, as emotion evaluation information, on the basis of the classification information; a storage step of associating the data element with the emotion evaluation information determined for the data element and storing them in a memory unit; and an unknown data evaluation step of evaluating an emotion of a user who has created unknown data, on the basis of the emotion evaluation information stored in the memory unit when new data is acquired as the unknown data.

12. A data evaluation program for having a computer implement:

an acquisition function that acquires, as training data, data including information representing an emotion of a user and classification information for classifying the emotion; an emotion evaluation function that determines a degree indicating how much a data element included in the training data reflects the user's emotion, as emotion evaluation information, on the basis of the classification information; a storage function that associates the data element with the emotion evaluation information determined for the data element and stores them in a memory unit; and an unknown data evaluation function that evaluates an emotion of a user who has created unknown data, on the basis of the emotion evaluation information stored in the memory unit when new data is acquired as the unknown data.

FIG.1

100

Communication
Unit  110

Display Unit  150

Input Unit  120

130

Control Unit

Presentation
Unit  139

Unclassified Data
Evaluation Unit  138

Evaluation Information
Acceptance Unit  132

Data Extraction
Unit  131

Data Classification
Unit  133

Element Extraction
Unit  134

Emotion Extraction
Unit  135

Emotion Evaluation
Unit  136

Evaluation Storage
Unit  137

Memory Unit  140

FIG.2

200

210　　　　　　　　　　　　230

Product Picture A　　　　　Product Information Field

◁ | Product Picture B | Product Picture C | Product Picture D | ▷　　220

241 — | Name: Anonymous
Evaluation: ☆☆☆☆☆
Comment: This is beautiful. It's also easy to use. | ∧

242 — | Name: Anonymous
Evaluation: ☆☆☆☆☆
Comment: Mediocre

243 — | Name: Fujimura
Evaluation: ☆☆☆☆☆
Comment: I'm happy it has XX function.

244 — | Name: Anne
Evaluation: ☆☆☆☆☆
Comment: It's kind of expensive. | ∨

FIG.3

```
                        ┌─────────────┐
                        │    START    │
                        └─────────────┘
                               │
                               ▼
        ┌──────────────────────────────────────┐
        │       Collect website information     │ ⌇ S301
        │      including evaluation information  │
        └──────────────────────────────────────┘
                               │
                               ▼
        ┌──────────────────────────────────────┐
        │          Classify them based on       │ ⌇ S302
        │           evaluation information      │
        └──────────────────────────────────────┘
                               │
                               ▼
        ┌──────────────────────────────────────┐
        │      Extract data elements from website│ ⌇ S303
        └──────────────────────────────────────┘
                               │
                               ▼
        ┌──────────────────────────────────────┐
        │        Extracts emotional expressions │ ⌇ S304
        │             from data elements        │
        └──────────────────────────────────────┘
                               │
                               ▼
        ┌──────────────────────────────────────┐
        │    Calculate evaluation of the extracted│ ⌇ S305
        │            emotional expressions      │
        │            based on classification    │
        └──────────────────────────────────────┘
                               │
                               ▼
        ┌──────────────────────────────────────┐
        │           Associate the extracted     │
        │        emotional expressions with the │ ⌇ S306
        │     calculated evaluation and store them│
        │              in memory unit           │
        └──────────────────────────────────────┘
                               │
                               ▼
                        ┌─────────────┐
                        │     END     │
                        └─────────────┘
```

FIG.4

```
        ┌──────────────┐
        │    START     │
        └──────┬───────┘
               │
               ▼
 ┌─────────────────────────────┐
 │       Input new data        │ ～S401
 └──────────────┬──────────────┘
                │
                ▼
 ┌─────────────────────────────┐
 │ Extract data elements from  │ ～S402
 │         the data            │
 └──────────────┬──────────────┘
                │
                ▼
 ┌─────────────────────────────┐
 │  Extract emotional          │
 │  expressions from the       │ ～S403
 │  extracted data elements    │
 └──────────────┬──────────────┘
                │
                ▼
 ┌─────────────────────────────┐
 │     Evaluate new data       │
 │   based on the evaluated    │ ～S404
 │   emotional expressions     │
 └──────────────┬──────────────┘
                │
                ▼
        ┌──────────────┐
        │     END      │
        └──────────────┘
```

## INTERNATIONAL SEARCH REPORT

| | International application No. |
|---|---|
| | PCT/JP2015/052777 |

**A. CLASSIFICATION OF SUBJECT MATTER**
*G06F17/30*(2006.01)i

According to International Patent Classification (IPC) or to both national classification and IPC

**B. FIELDS SEARCHED**

Minimum documentation searched (classification system followed by classification symbols)
G06F17/30

Documentation searched other than minimum documentation to the extent that such documents are included in the fields searched
Jitsuyo Shinan Koho          1922-1996   Jitsuyo Shinan Toroku Koho   1996-2015
Kokai Jitsuyo Shinan Koho    1971-2015   Toroku Jitsuyo Shinan Koho   1994-2015

Electronic data base consulted during the international search (name of data base and, where practicable, search terms used)
JSTPlus(JDreamIII)

**C. DOCUMENTS CONSIDERED TO BE RELEVANT**

| Category* | Citation of document, with indication, where appropriate, of the relevant passages | Relevant to claim No. |
|---|---|---|
| A | JP 2011-180988 A (Tokyo Institute of Technology), 15 September 2011 (15.09.2011), entire text; all drawings (Family: none) | 1-12 |
| A | WO 2011/079311 A1 (DUONG-VAN,MINH), 30 June 2011 (30.06.2011), entire text; all drawings & JP 2013-525868 A     & US 2011/0161071 A1 & US 2012/0101808 A1    & EP 2517156 A1 & CN 102812475 A | 1-12 |
| A | JP 2012-44562 A (Kyocera Corp.), 01 March 2012 (01.03.2012), entire text; all drawings (Family: none) | 1-12 |

☒ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 April 2015 (24.04.15) | 12 May 2015 (12.05.15) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (July 2009)

**INTERNATIONAL SEARCH REPORT**

International application No.

PCT/JP2015/052777

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2007-18234 A  (National Institute of Information and Communications Technology), 25 January 2007 (25.01.2007), entire text; all drawings (Family: none) | 1-12 |
| A | JP 2013-246636 A  (Nippon Telegraph and Telephone Corp.), 09 December 2013 (09.12.2013), entire text; all drawings (Family: none) | 1-12 |
| A | US 2006/0200341 A1  (MICROSOFT CORP.), 07 September 2006 (07.09.2006), entire text; all drawings & US 2006/0200342 A1    & US 7788086 B2 & US 7788087 B2 | 1-12 |

Form PCT/ISA/210 (continuation of second sheet) (July 2009)

**EP 3 089 053 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007018234 A